# EUROPEAN PATENT SPECIFICATION

(11) **EP 1 576 961 B1**
(45) Date of publication and mention of the grant of the patent: **07.11.2007**
(21) Application number: 03781099.1
(22) Date of filing: 11.12.2003
(51) Int. Cl.: A61K 36/28, A61P 1/18

(54) **USE OF SILYMARIN AND CARBOPOL AS A REGENERATOR OF PANCREATIC TISSUE AND CELLS WITH ENDOCRINE SECRETION DAMAGED BY DIABETES MELLITUS**
VERWENDUNG VON SILYMARIN UND CARBOPOL ZUR REGENERIERUNG VON PANKREASGEWEBE UND -ZELLEN MIT DURCH DIABETES MELLITUS BEEINTRÄCHTIGTER ENDOKRINER SEKRETION
UTILISATION DE SILYMARINE ET CARBOPOL COMME REGENERATEUR DU TISSU ET DES CELLULES PANCREATIQUES DE SECRETION ENDOCRINE ENDOMMAGES PAR LE DIABETE SUCRE

(30) Priority: 13.12.2002 MX PA02012315
(43) Date of publication of application: 21.09.2005
(73) Proprietor: Universidad Autonoma Metropolitana, C.P. 14387 México, D.F. (MX)
(72) Inventor: SOTO PEREDO, Claudia, Angélica, México, D.F., C.P. 01170170 (MX)
(74) Representative: Grünecker, Kinkeldey, Stockmair & Schwanhäusser Anwaltssozietät
(86) International application number: PCT/MX2003/000108
(87) International publication number: WO 2004/054600

(56) References cited:
- SOTO C. P. ET AL: "Prevention of alloxan-induced diabetes mellitus in the rat by silymarin." COMPARATIVE BIOCHEMISTRY AND PHYSIOLOGY, PART C: PHARMACOLOGY & ENDOCRINOLOGY , vol. 119c, num. 2, 1998, páginas 125-129, XP002906015 Elsevier Science Inc. ISSN: 0742-8413
- SOTO C. ET AL: "Silymarin regenerates pancreatic tissue damaged by alloxan in hyperglycemic rats." DIABETES & METABOLISM, 18TH INTERNATIONAL DIABETES FEDERATION CONGRESS ,PARIS, FRANCE, vol. 29 (hors serie 2), 24 - 29 Agosto 2003, página 4S157 XP002906016 ISSN: 1262-3636
- SCHOENFELD VON J ET AL: "SILIBININ, A PLANT EXTRACT WITH ANTIOXIDANT AND MEMBRANE STABILIZING PROPERTIES, PROTECTS EXOCRINE PANCREAS FROM CYCLOSPORINA TOXICITY" CMLS, CELLULAR AND MOLECULAR LIFE SCIENCES, BIRKHAUSER VERLAG, BASEL, CH, vol. 53, num. 11/12, Diciembre 1997 (1997-12), páginas 917-920, XP000965040 ISSN: 1420-682X
- DATABASE WPI Section Ch, Week 200205 Derwent Publications Ltd., London, GB; Class B04, AN 2002-037628 XP002906017 & JP 2001 261571 A (API KK), 26 Septiembre 2001 (2001-09-26)

## Description

### BACKGROUND OF THE INVENTION

Currently, in the pharmacological treatment of Diabetes Mellitus, there is use of insulin and groups of hypoglycemic agents such as the Sulfonylureas, the Biguanides, α-glucosidase inhibitors and Thiazolidinedione derivatives, whose action mechanism works by either stimulating the secretion of insulin or increasing the action of this hormone on the bodily tissues. The half-life of these drugs varies from 1.5 to 48 hours. Although all these drugs, by different action mechanisms, regulate the concentration of blood glucose and/or the secretion of insulin, the duration of their effect is a short period of time and none of them carries out the regeneration of the β-pancreatic cells that produce insulin and that allow the restoration of the function of this hormone. On the other hand, both exogenous insulin and hypoglycemic agents can cause side effects, such as: hypoglycemia, diarrhea, abdominal upsets, nausea and anorexia (Davis, S. and Granner, D., Insulin, Oral Hypoglycemic Agents and the Pharmacology of the Endogenous Pancreas. In: The Pharmacological Basis of Therapeutics. Eds.: Hardman, J.G., Limbird, L.E., Molinoff, P.B., Ruddon, R.W., Goodman, R., 8th Ed. pp. 1581-1614, 1996).
None of the medication used currently for the treatment of Diabetes Mellitus regenerates either damaged tissues or cells of endogenous secretion, including the β-pancreatic cells, as the Silymarin and Carbopol compound does. Both *in vitro* and *in vivo*, it has been demonstrated that Silymarin has a protective and regenerative activity on the hepatic cells against the damage produced by different toxic substances such as: phalloidin, α-amanitin, alcohol, galactosamine, heavy metals, carbon tetrachloride, toluene, xylene or by certain drugs such as: acetaminophen, indomethacin, isoniazid and tolbutamide (Wellington, K., Harvis, B., Silymarin: A review of its clinical properties in the management of hepatic disorders. Biodrugs. 15: 465-489, 2001) since it has been shown to stabilize the cellular membrane and protect it against free radicals, i.e., it has antioxidative properties by producing an increase in the hepatic glutathione content (free radical scavenger) (Valenzuela, A., Garrido, A. Biochemical basis of the pharmacological action of the flavonoid silymarin and of its structural isomer silibinin. Biol. Res. 27:105-112, 1994).

In humans, Silymarin has been used to treat hepatic diseases such as cirrhosis, poisoning by the fungus Amanita phalloides and exposure to toxic substances (Wellington, K., Harvis, B., Silymarin: A review of its clinical properties in the management of hepatic disorders. Biodrugs. 15: 465-489, 2001).

It has been suggested that free radicals play an important role in the etiology of Diabetes Mellitus, since high serum levels of malondialdehyde (end product of lipoperoxidation) in patients with this disease (Paolisso G, De Amore, A, Di Maro G, D'Onofrio F: Evidence for a relationship between free radicals and insulin action in the elderly. Metabolism, 42:659-66, 1993). Such levels of malondialdehyde are in direct relation to the degree of complications present in patients with Diabetes Mellitus. Defense mechanisms against free radicals include glutathione and antioxidant enzymes such as superoxide dismutase, glutathione peroxidase and catalase. Soto et al. (Soto C, Pérez B, Favari L, Reyes J: Prevention of alloxan-induced diabetes mellitus in the rat by silymarin. Comp. Biochem. Physiol. 119C:125-129, 1998) reported that Silymarin increases the pancreatic and hepatic glutathione content and therefore its level in blood.

The authors of the aforementioned work also found that Silymarin impeded the elevation of free radicals in the pancreas during the induction of Diabetes Mellitus, which led to a reduction of the blood glucose which is found to be high in this disease.

Taking into consideration the background described, the compound of Silymarin and Carbopol was used as an antidiabetic agent which it is sought to be protected through this application, since it presented an unknown effect as a pancreatic regenerator and controller of the serum concentration of the glucose, which is not regulated in diabetic patients.

### DESCRIPTION OF THE INVENTION

This invention corresponds to the use of Silymarin with Carbopol as a regenerator of the pancreatic tissue and cells of endogenous secretion damaged by Diabetes Mellitus, which suppresses the inconvenient side effects that are presented with the administration of drugs currently used in the treatment of Diabetes Mellitus.

The compound of Silymarin and Carbopol is obtained by the following steps:
a) Dissolution at 0.5 % of Carbopol in deionized water in a magnetic agitator for one hour.
b) Addition of Silymarin in a percentage of 5 to the foregoing dissolution and subjected to agitation for a minimum period of one hour until a homogenous mixture is obtained.

The homogenous mixture may be presented with any vehicle in formulations for oral administration, such as: solution, suspension, emulsion, hard gelatin capsules, soft gelatin capsules, immediate release tablets, sustained release tablets, prolonged release tablets, controlled release tablets.

An illustrative but not limitative example to demonstrate the pancreatic regenerative activity of the Silymarin and Carbopol compound is found in the one that was administered orally at a dose of 200 mg/Kg, either simultaneously with the diabetogenic agent Alloxan or separately.

The treatment of the Silymarin and Carbopol combined with Alloxan was done in two ways. In the first case, a dose of 200 mg/Kg of the Silymarin and

Carbopol compound was administered orally at 6, 24 and 48 hours (two days) after the first dose. In the second case, in addition to the dose mentioned in the first case, the administration was prolonged every 24 hours after the first dose for five more days (seven days). In both cases, the dose of Alloxan was 150 mg/Kg, which was administered subcutaneously one hour after the first dose of the Silymarin and Carbopol compound.

Another example that was also used to demonstrate the pancreatic regenerative activity of the Silymarin and Carbopol compound is in the one that proved the effect of this compound separately with the diabetogenic agent Alloxan, i.e. first the Alloxan was administered and then after 20 days the daily treatment with the Silymarin and Carbopol was begun at the same doses as those already mentioned in the first two cases above, for a period of time from 4 to 7 weeks.

The demonstration of the antidiabetic effect of this Silymarin and Carbopol compound is described in the examples mentioned below.

### Example 1

### Determination of serum glucose

The determination of blood glucose was carried out by the Baner method. This consisted in taking 50 µl of serum to which 3.0 ml of ortho-toluidine reagent were added which reacts with aldohexoses and forms glucosamine and a Schiff base, the green color developed has a maximum absorption at 620 nm and is proportional to the quantity of glucose present.

In the case of the combined administration of the Silymarin and Carbopol compound with Alloxan, the determination of serum glucose was done before the administration of the drugs and at 3, 5, 15 and 30 days after the treatment. For the case where the treatment of the Silymarin and Carbopol compound was begun 20 days after the administration of the Alloxan, the determination of the serum glucose was done before the administration of any drug, 20 days after the application of the Alloxan and after each week subsequent to the start of the treatment with the Silymarin and Carbopol compound.

The results showed that in Diabetes Mellitus (treatment with Alloxan only) there was an increase of between 100 and 400% in the serum levels of the glucose compared with the normal values, which range from 80 to 120 mg/dl. The Silymarin and Carbopol compound administered jointly with the diabetogenic agent Alloxan prevented the increase in the concentration of serum glucose that was presented with the administration of Alloxan alone. The results obtained from the treatment with the Silymarin and Carbopol compound started twenty days after the administration of the Alloxan showed that the values of the serum glucose concentration that were significantly high, approximately 400% above the normal value (before starting the treatment with the Silymarin and Carbopol compound) gradually decreased to reach the normal levels in an average time of seven weeks.

The treatment with the Silymarin and Carbopol compound administered alone did not modify the serum glucose concentration.

### Example 2

### Determination of the concentration of blood insulin by the ELISA method (Enzyme Linked Immuno Sorbent Assay).

This determination was carried out in 10 µl of serum, which was incubated for two hours with monoclonal antibodies aimed against separate antigenic determinants in the insulin molecule. During the incubation, the insulin of the sample reacted with anti-insulin antibodies and with peroxidase-conjugated anti-insulin antibodies. After this time, flushing was done to remove the antibody that failed to combine with the insulin molecules of the sample. 200 µl of 3,3,5,5'-tetramethylbenzidine was then added and the samples remain with this reagent for 30 minutes in order to be able to detect the conjugated compound, which is colored and can be quantified spectrophotometrically. The reaction was finalized by the addition of 50 µl of sulfuric acid and it was read at 450 nm in an ELISA reader in order to be able to determine the insulin concentration in each of the samples.

In the case of the combined administration of the Silymarin and Carbopol compound and Alloxan, the determination of serum insulin was done before the administration of the drugs and at 3, 5, 15 and 30 days after the treatment. For the case where the treatment of the Silymarin and Carbopol compound was begun 20 days after the administration of the Alloxan, the determination of the serum insulin was done before the administration of any drug, 20 days after the application of the Alloxan and at the end of the treatment with the Silymarin and Carbopol compound (when the glucose levels were found to be in the range of the normal values).

The results showed that in Diabetes Mellitus (treatment with Alloxan only) there was a decrease of between 80 and 90% in the serum levels of insulin compared with the normal value, which is 1 ηg/ml. It was observed that the joint treatment of the Silymarin and Carbopol compound and Alloxan impeded the reduction of the insulin serum levels and kept them within the range of the normal value. In the case of the treatment with the Silymarin and Carbopol 20 days after the administration of Alloxan, the insulin serum level was seen to increase to the range of the normal values. These normal values of serum insulin corresponded with those of glucose (example 1).

### Example 3:

### Histopathological analysis of the pancreatic tissue

This analysis was done in transversal fragments of pancreas approximately 0.7 cm long corresponding to the head of the organ. Subsequently, by the paraffin embedding technique, 5 µm sections where then obtained, which were stained with the dyes hematoxylin and eosin (HE). This technique consists in: 1) dehydration of the tissue, which is done by passing the tissue through different concentration of alcohol (from 25 to 100%) and then to an alcohol-toluene, toluene-paraffin and paraffin mixture. Once the tissues are dehydrated, blocks are formed with paraffin. 2) Sectioning of the tissue. This is done in a microtome, which is adjusted to the right section thickness of 4 to 6 µm and is placed in a slide. 3) Staining of the sections. This was done with the dyes hematoxylin and eosin.

In the case of the combined administration of the Silymarin and Carbopol compound and Alloxan, the histopathological analysis was done at 3, 5, 15 and 30 days after the treatment. For the case where the treatment of the Silymarin and Carbopol compound was begun 20 days after the administration of the Alloxan, the histopathological analysis was done 20 days after the application of the Alloxan and at the end of the treatment with the Silymarin and Carbopol compound (when the glucose levels were found to be in the range of the normal values, i.e. between 80 and 120 mg/dl).

In Diabetes Mellitus (treatment with Alloxan only) damage occurred in the pancreatic tissue that was observed as cellular disorganization with loss of the islets of Langerhans. Also observed were zones of necrosis, of cellular lysis, hemorrhagic zones, infiltration of lymphocytes, lysis of erythrocytes, as well as an increase in the adipose tissue that forms the capsule of the lobule.

The treatment with the Silymarin and Carbopol compound administered jointly with the Alloxan prevented the tissue damage in the pancreas that occurred in the Diabetes Mellitus, i.e. the tissue presented no alteration. The administration of the Silymarin and Carbopol compound, 20 days after the administration of the Alloxan, regenerated the pancreatic tissue, i.e. all the damage observed in the Diabetes Mellitus was completely reverted and a completely normal tissue was observed.

### Example 4

### Immunohistochemical analysis for insulin and glucagon in the islets of Langerhans by confocal microscopy

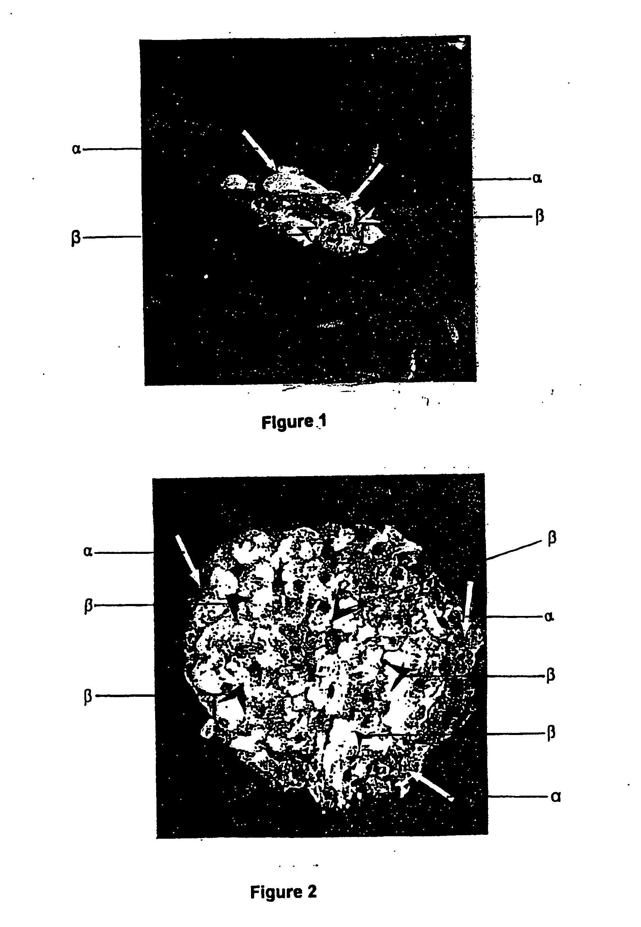

This analysis is carried out on sections of pancreatic tissue (corresponding to the head of the pancreas). Once the sections have been obtained (described in example 3) they are placed on a slide and the tissue is then deparaffinated and rehydrated. It is begun in pure xylol, with two changes of 10 minutes each. The samples are then passed to a mixture of ethanol-xylol for 10 minutes and afterward to alcohols of different percentages: 100, 90, 80 and 70% for five minutes each. Finally, they are passed to a PBS solution (phosphate buffered solution) for 30 minutes in order to then carry out the immunostaining process. This is begun with the incubation of the samples in a PBS-Triton solution for 10 minutes at an ambient temperature (20-22°C), they are flushed with PBS without triton and the samples are blocked with albumin in PBS. The samples are flushed with PBS for 1 to 5 minutes. A mixture is then made of primary antibodies anti-insulin and anti-glucagon in PBS and they are left to incubate for approximately 12 hours. At the end of this time, they are flushed with PBS and incubated in a mixture of secondary antibodies coupled with fluorochromes, fluorescein and rhodamine, for one hour at ambient temperature and they are then mounted in a specific medium to preserve the fluorescence. The samples are analyzed by confocal microscopy. In Diabetes Mellitus (treatment with Alloxan only) (figure 1) a destruction occurred of the pancreatic islet and only fragments of this were observed with a very scarce presence of insulin (β) y glucagon (α). In the treatment with the Silymarin and Carbopol compound administered jointly with the Alloxan (figure 2), the destruction of the pancreatic islet was prevented and this presented an image very similar to that of an islet in normal conditions, i.e. all the cells that form it were shown to be preserved. In most of these, insulin (β) is present, produced from the β cells and only in those of the periphery was the glucagon (α) observed, produced from the α cells.

The administration of the Silymarin and Carbopol compound twenty days after the Alloxan reversed the damage observed in the Diabetes Mellitus, i.e. it caused regeneration of the destroyed pancreatic islet presenting an islet with normal characteristics (described in the previous paragraph), which implies the recovery of the functioning of the β-pancreatic cells and therefore the production of insulin.

This methodology is equivalent to applying it on the human body on any type of Diabetes, since the action level of this composition is at the level of the dysfunction or destruction of the β pancreatic cells and their mechanisms against the cellular damage due to free radicals.

### Example 5:

### Determination of the activity of the pancreatic antioxidant enzymes

A determination was made of the pancreatic activity of the antioxidant enzymes: superoxide dismutase, glutathione peroxidase and catalase. To do such determination, the pancreas was weighed, it was homogenized with a phosphate buffer 50mM, it was centrifuged at 3000 rpm, and the supernatant was separated. The activities of the aforementioned enzymes was determined in this.

Superoxide dismutase.- This quantification was done by the method of Prasad et al. (1992). Superoxide ions are formed by xanthine and xanthine oxidase. These ions react with the tetrazolium blue and they form a colour compound that can be spectrophotometrically quantified at 560nm. A unit of superoxide dismutase can inhibit the formation of this compound by 50%.

Glutathione peroxidase.- This quantification was done by the method of Prasad et al. (1992). This enzyme catalyzes the breakdown of H₂O₂ by the NADPH. Its activity is measured by the velocity of absorbance change during the conversion of NADPH to NADP⁺ and this may be spectrophotometrically registered at 300 nm.

Catalase.- This quantification was carried out by the Aebi method (1995). This enzyme catalyzes the breakdown of H₂O₂ and the decomposition velocity of the oxygenated water is measured spectrophotometrically at 240nm.

In the Diabetes Mellitus (treatment with Alloxan only) there was a decrease of between 70 and 75% in the activity of the three enzymes studied: superoxide dismutase, glutathione peroxidase and catalase.

The combined administration of the Silymarin and Carbopol compound and

Alloxan showed an increase in the activity of the pancreatic antioxidant enzymes, since this was no different than the control (normal) values.

The administration of the Silymarin and Carbopol compound twenty days after the Alloxan reverted the damage observed in the Diabetes Mellitus in the activity of the three enzymes studied and even values of enzymatic activities higher than normal values were observed (see table).

| Enzymatic activity/Treatment | Alloxan (20 days after its administration) | Silymarin administered 20 days after the application of the Alloxan |
|---|---|---|
| Glutathione Peroxidase µM NADPH/min/mg prot | 0.016 ±.002 | 0.052±0.002 |
| Superoxide Dismutase U/mg protein. | 4.3±0.02 | 46.5±0.50 |
| Catalase k/sec/mg of protein | 0.01±0.002 | 0.04±0.00 |

## Claims

1. The use of a composition containing Silymarin and Carbopol for the manufacture of a medicine for regenerating damaged pancreatic cells and for recovering the endocrine pancreatic function, wherein the medicament comprises a pharmaceutically acceptable vehicle.

2. The use in accordance with claim 1, wherein the composition contains 3 to 7% Silymarin and 0.2 to 0.6% Carbopol.

3. The use in accordance with claim 2, wherein the composition contains 5% Silymarin and 0.5% Carbopol.

4. The use in accordance with any of claims 1 to 3, wherein the pharmaceutical composition is in the form of an oral dose.

5. The use in accordance with claim 4, wherein the oral form is an oral suspension, oral solution, emulsion, gel, hard gelatin capsule, soft gelatin capsule, immediate release tablet, controlled release tablet, prolonged release or sustained release tablet.

6. The use in accordance with claim 5, wherein the oral form is an oral suspension.

7. The use in accordance with any of claims 1 to 6, wherein the functioning of the β-pancreatic cells causes the production of insulin.

8. The use in accordance any of claims 1 to 7, wherein the medicine is useful for the treatment of diabetes mellitus.

9. The use according to claims 1, 7 and 8, wherein the dosage for administration is from 60 to 220 mg/kg.

10. The use according to claim 9, wherein the dosage for administration is 220 mg/kg.

## Patentansprüche

1. Verwendung einer Silymarin und Carbopol enthaltenden Zusammensetzung zur Herstellung eines Medikaments zum Regenerieren geschädigter Pankreaszellen und zur Wiederherstellung der endokrinen Pankreasfunktion, worin das Medikament einen pharmazeutisch annehmbaren Träger enthält.

2. Verwendung gemäß Anspruch 1, worin die Zusammensetzung 3 bis 7 % Silymarin und 0,2 bis 0,6 % Carbopol enthält.

3. Verwendung gemäß Anspruch 2, worin die Zusammensetzung 5 % Silymarin und 0,5 % Carbopol enthält.

4. Verwendung gemäß einem der Ansprüche 1 bis 3, worin die pharmazeutische Zusammensetzung in Form einer oralen Dosierung vorliegt.

5. Verwendung gemäß Anspruch 4, worin die orale Form eine orale Suspension, eine orale Lösung, eine Emulsion, ein Gel, eine Hartgelatinekapsel, eine Weichgelatinekapsel, eine Tablette mit unmittelbarer Freisetzung, eine Tablette mit geregelter Freisetzung, eine Tablette mit verlängerter Freisetzung oder eine Tablette mit Depotwirkung ist.

6. Verwendung gemäß Anspruch 5, worin die orale Form eine orale Suspension ist.

7. Verwendung gemäß einem der Ansprüche 1 bis 6, worin das Wirken der β-Pankreaszellen die Produktion von Insulin hervorruft.

8. Verwendung gemäß einem der Ansprüche 1 bis 7, worin das Medikament zur Behandlung von Diabetes mellitus verwendbar ist.

9. Verwendung gemäß den Ansprüchen 1, 7 und 8, worin die Verabreichungsdosis 60 bis 220 mg/kg beträgt.

10. Verwendung gemäß Anspruch 9, worin die Verabreichungsdosis 220 mg/kg beträgt.

## Revendications

1. Utilisation d'une composition contenant de la silymarine et du carbopol pour la fabrication d'un médicament destiné à régénérer des cellules pancréatiques endommagées et à rétablir la fonction pancréatique endocrine, dans laquelle le médicament comprend un véhicule pharmaceutiquement acceptable.

2. Utilisation selon la revendication 1, dans laquelle la composition contient 3 à 7 % de silymarine et 0,2 à 0,6 % de carbopol.

3. Utilisation selon la revendication 2, dans laquelle la composition contient 5 % de silymarine et 0,5 % de carbopol.

4. Utilisation selon l'une quelconque des revendications 1 à 3, dans laquelle la composition pharmaceutique est sous la forme d'une dose orale.

5. Utilisation selon la revendication 4, dans laquelle la forme orale est une suspension orale, une solution orale, une émulsion, un gel, une capsule de gélatine dure, une capsule de gélatine molle, un comprimé à libération immédiate, un comprimé à libération contrôlée, un comprimé à libération prolongée ou à libération continue.

6. Utilisation selon la revendication 5, dans laquelle la forme orale est une suspension orale.

7. Utilisation selon l'une quelconque des revendications 1 à 6, dans laquelle le fonctionnement des cellules β-pancréatiques provoque la production d'insuline.

8. Utilisation selon l'une quelconque des revendications 1 à 7, dans laquelle le médicament est utile pour le traitement du diabète sucré.

9. Utilisation selon les revendications 1, 7 et 8, dans laquelle le dosage pour l'administration est compris entre 60 et 220 mg/kg.

10. Utilisation selon la revendication 9, dans laquelle le dosage pour l'administration est de 220 mg/kg.
